# EUROPEAN PATENT APPLICATION

(11) **EP 4 488 380 A1**
(43) Date of publication of application: **08.01.2025**
(21) Application number: 23779419.3
(22) Date of filing: 10.03.2023
(51) Int. Cl.: C12Q 1/02, C12M 1/00, C12M 1/34

(54) **CELL CULTURE DEVICE AND CALIBRATION METHOD**

(30) Priority: 28.03.2022 JP 2022051961
(71) Applicant: Terumo Kabushiki Kaisha, Tokyo 151-0072 (JP)
(72) Inventor: OKUBO, Itaru, Ashigarakami-gun, Kanagawa 259-0151 (JP); LIU, Liming, Ashigarakami-gun, Kanagawa 259-0151 (JP)
(74) Representative: TBK
(86) International application number: PCT/JP2023/009203
(87) International publication number: WO 2023/189395

(57) **Abstract**

A cell culture device (10) includes a cell culture circuit (16), a sensor (130) that is disposed in the cell culture circuit and measures turbidity of a cell fluid, and a calibration unit (126). The calibration unit (126) calculates, as a first concentration, a cell concentration of a culture medium in which the cell fluid is supplied, calculates, as a second concentration, a cell concentration of the diluted culture medium, and creates a calibration curve between a measurement value of the sensor and the cell concentration by associating the first concentration with the first measurement value and associating the second concentration with the second measurement value.

## Description

### Technical Field

The present invention relates to a cell culture device having a sensor calibration function and a sensor calibration method.

### Background Art

JP 2015-50983 A discloses a cell culture device for culturing cells. This cell culture device uses a turbidity sensor as a sensor for measuring the cell concentration of the culture medium. The turbidity sensor measures, for example, transmitted light, scattered light, and the like from light applied to the solution. The higher the cell concentration, the greater the number of cells in the culture medium. Then, the turbidity increases.

Turbidity is affected by cell size. For example, when comparing a culture medium A containing small cells with a culture medium B containing large cells, in a case where the numbers of both cells are the same, the light transmittance of the culture medium A is higher than the light transmittance of the culture medium B. That is, while the concentration of the culture medium A and the concentration of the culture medium B are the same, the turbidity of the culture medium A is lower than the turbidity of the culture medium B. The relationship between the turbidity and the cell concentration varies depending on the type of cells. Therefore, in a case where the cell concentration is measured by the turbidity sensor, it is necessary to set the relationship between the cell concentration and the turbidity in advance. For example, a calibration curve is set as a relationship between the cell concentration and the turbidity.

### Summary of Invention

Calibration work is required to set the calibration curve. In the conventional calibration work, for example, it is necessary to prepare two cell fluids having different cell concentrations. Further, the turbidity sensors were attached to the cell culture device after the turbidity sensors measured the turbidity of the two cell fluids individually.

According to the conventional calibration work, since the two types of cell fluid and culture medium were discarded after the calibration work, waste of the cell fluid and the culture medium occurred. In addition, the conventional calibration work requires work other than the cell culture device, and thus lacks convenience.

An object of the present invention is to solve the above-described problem.

A first aspect of the present invention is a cell culture device including a cell culture circuit capable of circulating a culture medium in which a cell fluid is supplied, a sensor that is disposed in the cell culture circuit and measures turbidity of the cell fluid, and a calibration unit that calibrates the measurement value of the sensor to a cell concentration of the culture medium, in which the sensor measures, as a first measurement value, the turbidity of a first volume of the culture medium in which the cell fluid containing a predetermined number of cells is supplied, and measures, as a second measurement value, the turbidity of the culture medium generated by diluting the first volume of the culture medium with a second volume of the culture medium not containing cells, and the calibration unit calculates, as a first concentration, the cell concentration of the culture medium in which the cell fluid is supplied, calculates, as a second concentration, the cell concentration of the culture medium after dilution, and creates a calibration curve between the measurement value of the sensor and the cell concentration by associating the first concentration with the first measurement value and associating the second concentration with the second measurement value.

A second aspect of the present invention is a calibration method using a cell culture circuit capable of circulating a culture medium in which a cell fluid is supplied, a sensor that is disposed in the cell culture circuit and measures turbidity of the cell fluid, and a calibration unit that calibrates the measurement value of the sensor to a cell concentration of the culture medium, the method including causing the sensor to measure, as a first measurement value, the turbidity of a first volume of the culture medium in which the cell fluid containing a predetermined number of cells is supplied, and measure, as a second measurement value, the turbidity of the culture medium generated by diluting the first volume of the culture medium with a second volume of the culture medium not containing cells, and causing the calibration unit to calculate, as a first concentration, the cell concentration of the culture medium in which the cell fluid is supplied, calculate, as a second concentration, the cell concentration of the culture medium after dilution, and create a calibration curve between the measurement value of the sensor and the cell concentration by associating the first concentration with the first measurement value and associating the second concentration with the second measurement value.

According to the present invention, it is possible to prepare a cell fluid in which the number of cells is known in advance and perform calibration using a cell culture device without performing an operation outside the cell culture device. Therefore, it is possible to improve the efficiency of the operation and to eliminate discarding of the cell fluid and the culture medium.

### Brief Description of Drawings

Fig. 1 is a diagram illustrating a configuration of a cell culture device.
Fig. 2 is a diagram illustrating functional blocks of an arithmetic unit.
Fig. 3 is a diagram illustrating a configuration of a sensor unit of a first embodiment.
Figs. 4A and 4B are diagrams illustrating a connection state between a first circulation flow path and a dilution flow path in the sensor unit of the first embodiment.
Fig. 5 is a flowchart of calibration processing according to the first embodiment.
Fig. 6 is a diagram illustrating a calibration curve.
Fig. 7 is a diagram illustrating a configuration of a sensor unit of a second embodiment.
Figs. 8A and 8B are diagrams illustrating a connection state between a first circulation flow path and a dilution flow path in the sensor unit of the second embodiment.
Fig. 9 is a diagram illustrating a configuration of a sensor unit of a third embodiment.
Fig. 10 is a diagram illustrating a configuration of a cell culture device (calibration device) according to another embodiment of Fig. 1.
Fig. 11 is a diagram illustrating a configuration of a sensor unit of a fourth embodiment.
Fig. 12A to Fig. 12D are diagrams illustrating a connection state of a first circulation flow path, a sensor flow path, and a concentration flow path in a sensor unit of a fourth embodiment.
Fig. 13 is a flowchart of calibration processing according to the fourth embodiment.
Fig. 14 is a diagram illustrating a configuration of a sensor unit of a fifth embodiment.
Fig. 15 is a diagram illustrating a configuration of a sensor unit of a sixth embodiment.
Fig. 16A to Fig. 16D are diagrams illustrating a connection state between a first circulation flow path, a sensor flow path, a concentration flow path, and a dilution flow path in the sensor unit of the sixth embodiment.
Fig. 17 is a flowchart of calibration processing according to the sixth embodiment.

### Description of Embodiments

### [1 Configuration of Cell Culture Device 10]

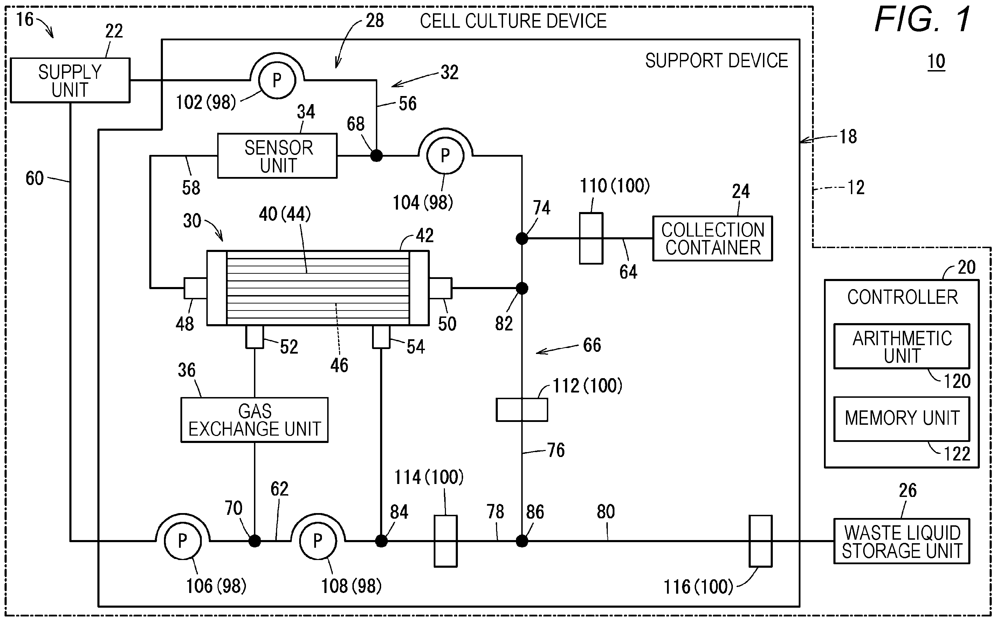

Fig. 1 is a diagram illustrating a configuration of a cell culture device 10 (calibration device 12). The calibration device 12 according to the present embodiment calibrates the measurement value of a sensor unit 34 using the cell culture device 10 for culturing cells. Therefore, first, a basic configuration of the cell culture device 10 will be described.

The cell culture device 10 cultures the cells separated from the biological tissue in a culture medium. The cells used in the cell culture device 10 are, for example, adherent cells or floating cells. Specifically, examples of the cells used in the cell culture device 10 include ES cells, iPS cells, mesenchymal stem cells, and the like. The cells used in the cell culture device 10 are not limited to those described above.

The cell culture device 10 includes a cell culture circuit 16, a support device 18, and a controller 20. A liquid flows in the cell culture circuit 16. The liquid includes at least one of a cell fluid, a culture medium, a washing liquid, and a stripping liquid.

The cell fluid is a solution containing cells. The culture medium is a culture solution for growing cells. The culture medium is selected according to the cell to be cultured. As the culture medium, for example, minimum essential media (MEM) are used. The washing liquid washes the inside of the cell culture circuit 16. As the washing liquid, for example, water, a buffer solution, physiological saline, or the like is used. Examples of the buffer solution include phosphate buffered salts (PBS), tris-buffered saline (TBS), and the like. The stripping liquid detaches cells from a bioreactor 30 of the cell culture circuit 16. As the stripping liquid, for example, trypsin, an EDTA solution, or the like is used. The culture medium, the washing liquid, and the stripping liquid are not limited to the liquid described above.

### [1-1 Cell Culture Circuit 16]

The cell culture circuit 16 is discarded after a single use. In other words, the cell culture circuit 16 is discarded every time a predetermined number of cells are cultured. That is, the cell culture circuit 16 is a disposable product. The cell culture circuit 16 includes a supply unit 22, a collection container 24, a waste liquid storage unit 26, and a culture body 28.

The supply unit 22 supplies the cell fluid, the culture medium, the washing liquid, and the stripping liquid to the culture body 28. Each liquid is filled in the medical bag. The collection container 24 collects the cells cultured in the culture body 28. The waste liquid storage unit 26 stores the waste liquid generated in the culture body 28. Each of the collection container 24 and the waste liquid storage unit 26 is, for example, a medical bag. Each of the collection container 24 and the waste liquid storage unit 26 may be a tank or the like made of a hard material.

The culture body 28 includes a bioreactor 30, a flow path 32, a sensor unit 34, and a gas exchange unit 36.

The bioreactor 30 has a plurality of hollow fiber membranes 40 and a cylindrical housing 42. The plurality of hollow fiber membranes 40 are stored in the housing 42. One end portion of each hollow fiber membrane 40 is fixed to one end portion of the housing 42. The other end of each hollow fiber membrane 40 is fixed to the other end of the housing 42. Each hollow fiber membrane 40 is made of, for example, a polymer material.

The bioreactor 30 includes a first region 44 and a second region 46. The first region 44 is an inner hole of the plurality of hollow fiber membranes 40. The second region 46 is a space between the inner peripheral surface of the housing 42 and the outer peripheral surfaces of the plurality of hollow fiber membranes 40. Each hollow fiber membrane 40 has a plurality of pores (not illustrated). The first region 44 and the second region 46 communicate with each other through the plurality of pores of each hollow fiber membrane 40. The diameter of each pore is a size that allows passage of small molecules (for example, water, ions, oxygen, lactate, and the like) while blocking passage of macromolecules (cells and the like). The diameter of each pore is set to, for example, 0.005 [um] or more and 10 [pm] or less.

A first inlet port 48, a first outlet port 50, a second inlet port 52, and a second outlet port 54 are attached to the housing 42. The first inlet port 48 is attached to one end of the housing 42. The first inlet port 48 communicates with the first region 44 via an inlet located at one end of the plurality of hollow fiber membranes 40. The first outlet port 50 is attached to the other end of the housing 42. The first outlet port 50 communicates with the first region 44 via an outlet located at the other end of the plurality of hollow fiber membranes 40.

The second inlet port 52 and the second outlet port 54 are attached to the outer peripheral surface of the housing 42. The second inlet port 52 is located between the center of the housing 42 and the first inlet port 48 in the longitudinal direction of the housing 42. The second outlet port 54 is located between the center of the housing 42 and the first outlet port 50 in the longitudinal direction of the housing 42. Each of the second inlet port 52 and the second outlet port 54 communicates with the second region 46.

The flow path 32 includes a plurality of tubes through which a liquid flows. Each tube is made of a soft resin material. The flow path 32 includes a first supply flow path 56, a first circulation flow path 58, a second supply flow path 60, a second circulation flow path 62, a collection flow path 64, and a waste liquid flow path 66. One end of the first supply flow path 56 is connected to the supply unit 22. The other end of the first supply flow path 56 is connected to a first junction portion 68 of the first circulation flow path 58.

The first junction portion 68 is located at an intermediate part of the first circulation flow path 58 in the extending direction. One end of the first circulation flow path 58 is connected to the first inlet port 48. The other end of the first circulation flow path 58 is connected to the first outlet port 50. The first circulation flow path 58 communicates with inner holes (first region 44) of the plurality of hollow fiber membranes 40.

One end of the second supply flow path 60 is connected to the supply unit 22. The supply unit 22 supplies the culture medium and the washing liquid one by one to the second supply flow path 60 at a predetermined timing. The other end of the second supply flow path 60 is connected to a second junction portion 70 of the second circulation flow path 62.

The second junction portion 70 is located at an intermediate part of the second circulation flow path 62 in the extending direction. One end of the second circulation flow path 62 is connected to the second inlet port 52. The other end of the second circulation flow path 62 is connected to the second outlet port 54. The second circulation flow path 62 communicates with a space (second region 46) between the plurality of hollow fiber membranes 40 and housing 42.

The supply unit 22 supplies the cell fluid, the culture medium, the washing liquid, and the stripping liquid one by one to the first circulation flow path 58 via the first supply flow path 56 at a predetermined timing. In addition, the supply unit 22 supplies the culture medium to the second circulation flow path 62 via the second supply flow path 60 at a predetermined timing.

The collection flow path 64 extends from the first circulation flow path 58. One end of the collection flow path 64 is connected to a collection branch portion 74 of the first circulation flow path 58. The collection branch portion 74 is positioned between the first junction portion 68 and the first outlet port 50 in the first circulation flow path 58. The other end of the collection flow path 64 is connected to the collection container 24.

A liquid to be discarded flows through the waste liquid flow path 66 from the first circulation flow path 58 and the second circulation flow path 62. The waste liquid flow path 66 includes a first waste liquid flow path 76, a second waste liquid flow path 78, and a third waste liquid flow path 80. The first waste liquid flow path 76 extends from the first circulation flow path 58. One end of the first waste liquid flow path 76 is connected to a first branch portion 82 of the first circulation flow path 58. The first branch portion 82 is positioned between the first outlet port 50 of the first circulation flow path 58 and the collection branch portion 74. The second waste liquid flow path 78 extends from the second circulation flow path 62. One end of the second waste liquid flow path 78 is connected to the second branch portion 84 of the second circulation flow path 62. The second branch portion 84 is located between the second junction portion 70 of the second circulation flow path 62 and the second outlet port 54. The other end of the first waste liquid flow path 76 and the other end of the second waste liquid flow path 78 are connected to each other at an intermediate junction portion 86. One end of the third waste liquid flow path 80 is connected to the first waste liquid flow path 76 and the second waste liquid flow path 78 at the intermediate junction portion 86. The other end of the third waste liquid flow path 80 is connected to the waste liquid storage unit 26.

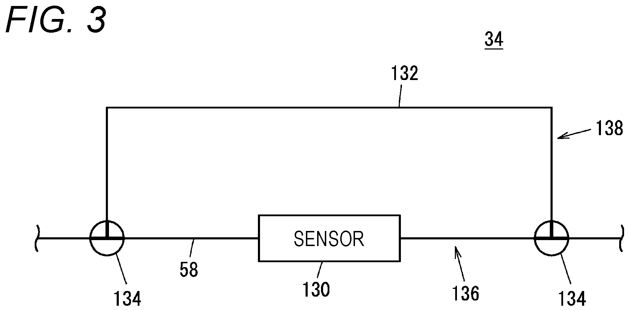

The sensor unit 34 is provided between the first junction portion 68 of the first circulation flow path 58 and the first inlet port 48. The sensor unit 34 includes a sensor 130 (Fig. 3 and the like) for turbidity measurement. The relationship between the measurement value of the sensor 130 and the true value of the turbidity is preset. The turbidity measurement value measured by the sensor 130 is converted into the cell concentration by a calibration unit 126 that will be described later. A specific configuration of the sensor unit 34 will be described later.

The gas exchange unit 36 is attached between the second junction portion 70 of the second circulation flow path 62 and the second inlet port 52. The gas exchange unit 36 allows a gas of a predetermined component to pass through a liquid (culture medium) flowing through the second circulation flow path 62. The gas used in the gas exchange unit 36 has, for example, similar components to air in nature. In other words, the gas includes nitrogen, oxygen, and carbon dioxide. Specifically, the gas contains, for example, 75% nitrogen, 20% oxygen, and 5% carbon dioxide in volume ratio.

### [1-2 Support Device 18]

The cell culture circuit 16 described above is set in the support device 18. The support device 18 has a cassette that supports the cell culture circuit 16. The support device 18 is a reusable product that can be used a plurality of times.

The support device 18 includes a plurality of pumps 98 and a plurality of clamps 100. Each of the plurality of pumps 98 applies a flow force to the liquid in the flow path 32 by squeezing the wall of the flow path 32. Each of the plurality of pumps 98 includes a pressing member (not illustrated). The pressing member includes, for example, a rotating member and a plurality of pressing rollers. The plurality of pressing rollers are attached to an outer peripheral portion of the rotating member. The plurality of pressing rollers are arranged at intervals in the circumferential direction of the rotating member. Each pressing roller rubs the outer surface of the wall portion of the flow path 32.

The plurality of pumps 98 include a first supply pump 102, a first circulation pump 104, a second supply pump 106, and a second circulation pump 108. As illustrated in Fig. 1, a state in which the cell culture circuit 16 is set in the support device 18 is simply referred to as a "set state".

In the set state, a part of the first supply flow path 56 is attached to the first supply pump 102. The first supply pump 102 applies a flow force in a direction from the supply unit 22 toward the first circulation flow path 58 to the liquid in the first supply flow path 56.

In the set state, a part of first circulation flow path 58 is attached to first circulation pump 104. The first circulation pump 104 applies a flow force in a direction from the first outlet port 50 toward the first inlet port 48 to the liquid in the first circulation flow path 58. The first circulation pump 104 can also apply a flow force in a direction from the first inlet port 48 toward the first outlet port 50 to the liquid in the first circulation flow path 58.

In the set state, a part of the second supply flow path 60 is attached to the second supply pump 106. The second supply pump 106 applies a flow force in a direction from the supply unit 22 toward the second circulation flow path 62 to the liquid in the second supply flow path 60.

In the set state, a part of the second circulation flow path 62 is attached to the second circulation pump 108. The second circulation pump 108 applies a flow force in a direction from the second outlet port 54 toward the second inlet port 52 to the liquid in the second circulation flow path 62. The second circulation pump 108 can also apply a flow force in a direction from the second inlet port 52 toward the second outlet port 54 to the liquid in the second circulation flow path 62.

The plurality of clamps 100 close the flow path 32 by pressing the outer surface of the flow path 32 toward the inner surface. For example, the plurality of clamps 100 are on-off valves. The plurality of clamps 100 include a collection clamp 110, a first waste liquid clamp 112, a second waste liquid clamp 114, and a third waste liquid clamp 116.

In the set state, a part of the collection flow path 64 is attached to the collection clamp 110. The collection clamp 110 opens and closes the collection flow path 64. In the set state, a part of the first waste liquid flow path 76 is attached to the first waste liquid clamp 112. The first waste liquid clamp 112 opens and closes the first waste liquid flow path 76. In the set state, a part of the second waste liquid flow path 78 is attached to the second waste liquid clamp 114. The second waste liquid clamp 114 opens and closes the second waste liquid flow path 78. In the set state, a part of the third waste liquid flow path 80 is attached to the third waste liquid clamp 116. The third waste liquid clamp 116 opens and closes the third waste liquid flow path 80.

### [1-3 Controller 20]

The controller 20 is, for example, a computer. The controller 20 includes an arithmetic unit 120, a memory unit 122, and various drive circuits (not illustrated).

The arithmetic unit 120 includes a processing circuit. The processing circuit may be a processor such as a CPU. The processing circuit may be an integrated circuit such as an ASIC or an FPGA. The processor can execute various types of processing by executing a program stored in the memory unit 122. At least some of the plurality of processing may be performed by an electronic circuit including a discrete device.

The memory unit 122 includes a volatile memory and a nonvolatile memory. Examples of the volatile memory include a RAM and the like. The volatile memory is used as a working memory of the processor. The volatile memory temporarily stores data and the like necessary for processing or computation. Examples of the nonvolatile memory include a ROM and a flash memory. The non-volatile memory is used as a storage memory. The nonvolatile memory stores programs, tables, maps, and the like. At least a part of the memory unit 122 may be provided in the processor, the integrated circuit, or the like as described above.

### [2 Calibration Device 12]

A configuration necessary in a case where the cell culture device 10 is used as the calibration device 12 will be described. The calibration device 12 uses the supply unit 22, the first supply flow path 56, the first supply pump 102, the first circulation flow path 58, the first circulation pump 104, the bioreactor 30, the sensor unit 34, and the controller 20 in each component of the cell culture device 10.

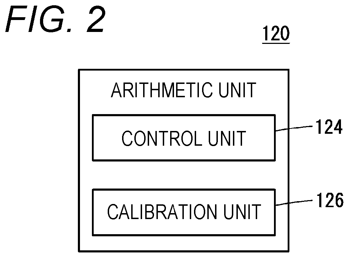

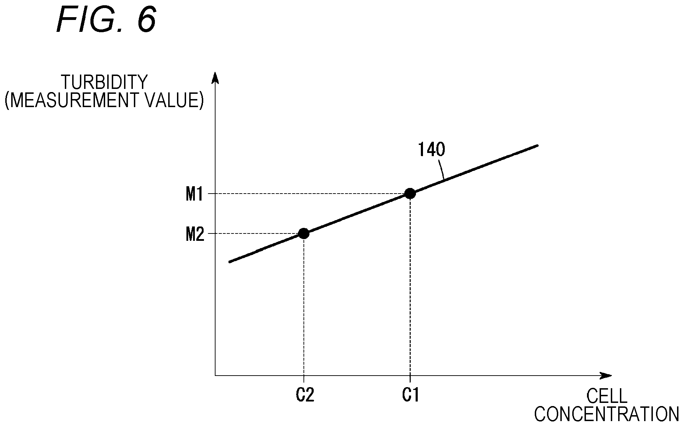

Fig. 2 is a diagram illustrating functional blocks of the arithmetic unit 120. The arithmetic unit 120 of the controller 20 functions as the control unit 124 and the calibration unit 126. The control unit 124 controls the first supply pump 102, the first circulation pump 104, and valves 134 to be described later. The calibration unit 126 acquires the measurement value of the turbidity from the sensor 130 and creates a calibration curve 140 (Fig. 6) between the measurement value of the turbidity and the cell concentration.

### [3 First Embodiment]

### [3-1 Configuration of Sensor Unit 34]

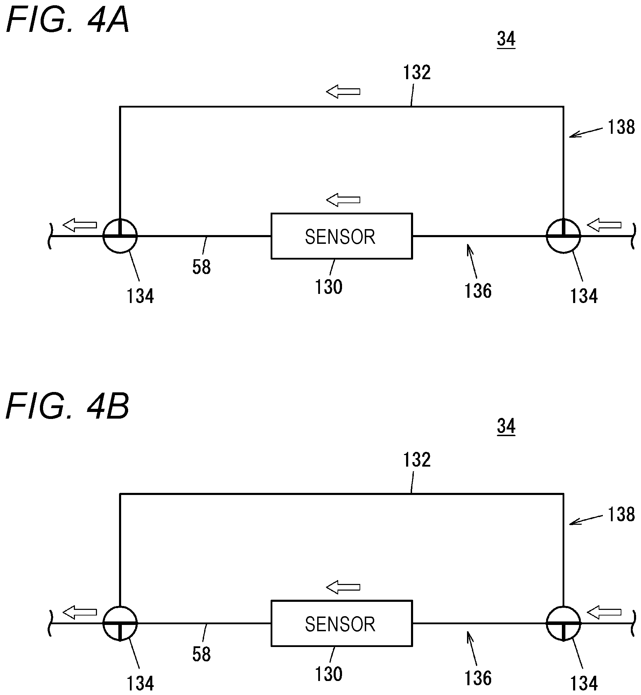

Fig. 3 is a diagram illustrating a configuration of the sensor unit 34 of the first embodiment. Figs. 4A and 4B are diagrams illustrating a connection state between the first circulation flow path 58 and the dilution flow path 132 in the sensor unit 34 of the first embodiment. In the first embodiment, the sensor unit 34 includes the first circulation flow path 58, the sensor 130, the dilution flow path 132, and the two valves 134.

The sensor 130 is a turbidity sensor. The sensor 130 is connected to the first circulation flow path 58 in series. The dilution flow path 132 is a tube member. The dilution flow path 132 is connected to sensor 130 of first circulation flow path 58 in parallel. The volume of the dilution flow path 132, the volume of the first circulation flow path 58, and the volume of the bioreactor 30 are stored in the memory unit 122 in advance. For example, the volume of the dilution flow path 132 is equal to the sum of the volume of the first circulation flow path 58 and the volume of the bioreactor 30. The valve 134 is disposed at each connection point between the first circulation flow path 58 and the dilution flow path 132.

The two valves 134 are, for example, three-way valves. As illustrated in Fig. 4A, the two valves 134 can communicate the first circulation flow path 58 and the dilution flow path 132 with each other. As illustrated in Fig. 4B, the two valves 134 can block the first circulation flow path 58 and the dilution flow path 132 from each other. The two valves 134 operate according to a command signal output from the control unit 124.

In the first embodiment, the first circuit 136 is formed by a closed circuit including the first circulation flow path 58, the sensor 130, the bioreactor 30 (Fig. 1), and the first circulation pump 104 (Fig. 1). Furthermore, in the first embodiment, the second circuit 138 is formed by the dilution flow path 132 connected to the first circuit 136 in parallel.

### [3-2 Calibration Processing]

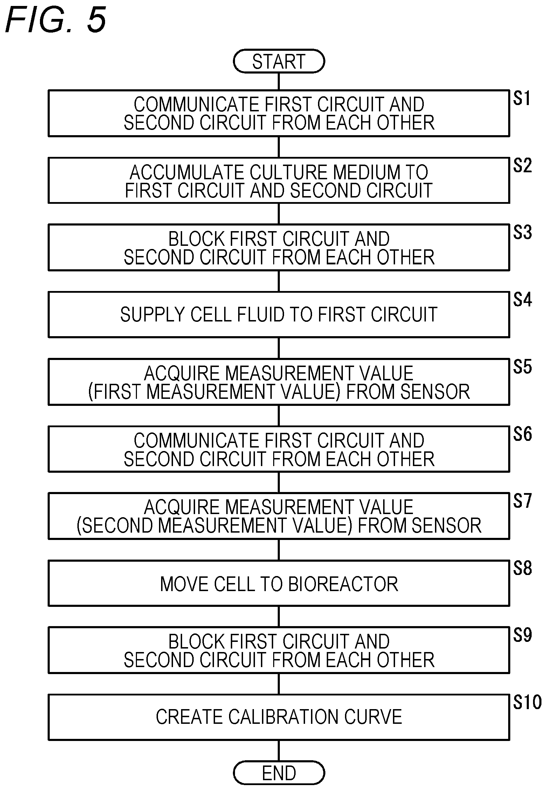

Fig. 5 is a flowchart of calibration processing according to the first embodiment. Before the calibration processing illustrated in Fig. 5, the user counts the number of cells in the cell fluid by a cell counter or the like. The user fills a medical bag or the like with the cell fluid of which the number of cells has been counted, and sets the medical bag in the supply unit 22. In addition, the user fills the medical bag with a culture medium not containing cells, and sets the medical bag in the supply unit 22. The user saves the counted number of cells in the memory unit 122.

In step S1, the control unit 124 causes the dilution flow path 132 and the first circulation flow path 58 to communicate with each other by controlling the valve 134 (Fig. 4A). As a result, the first circuit 136 and the second circuit 138 communicate with each other.

In step S2, the control unit 124 connects the first supply flow path 56 to the medical bag of the culture medium. The control unit 124 then operates the first supply pump 102 and the first circulation pump 104. According to the operations of the first supply pump 102 and the first circulation pump 104, the supply unit 22 supplies the culture medium containing no cells to the first circulation flow path 58. As a result, the culture medium not containing cells accumulates in both the first circuit 136 and the second circuit 138. After supplying the culture medium, the control unit 124 stops the first supply pump 102.

In step S3, the control unit 124 blocks the dilution flow path 132 and the first circulation flow path 58 from each other by controlling the valve 134 (Fig. 4B). As a result, the first circuit 136 and the second circuit 138 are blocked from each other.

In step S4, the control unit 124 connects the first supply flow path 56 to the medical bag of the cell fluid. The control unit 124 then operates the first supply pump 102 and the first circulation pump 104. The supply unit 22 supplies the cell fluid to the first circulation flow path 58 according to the operations of the first supply pump 102 and the first circulation pump 104. At this point, the dilution flow path 132 is blocked from the first circulation flow path 58. Therefore, in the sensor unit 34, the cell fluid is supplied only to the first circuit 136. The control unit 124 circulates the cell fluid for a predetermined time after the supply of the cell fluid is completed. After the cell fluid becomes uniform, that is, after the cell fluid is stabilized, the control unit 124 stops the first supply pump 102.

In step S5, the calibration unit 126 acquires the measurement value from the sensor 130. This measurement value is referred to as a first measurement value M1. The memory unit 122 saves the first measurement value M1.

In step S6, the control unit 124 causes the dilution flow path 132 and the first circulation flow path 58 to communicate with each other by controlling the valve 134 (Fig. 4A). As a result, the first circuit 136 and the second circuit 138 communicate with each other. Then, the culture medium of the second circuit 138 and the culture medium of the first circuit 136 are mixed. As a result, the culture medium containing the cells is diluted.

In step S7, the calibration unit 126 acquires the measurement value from the sensor 130. This measurement value is referred to as a second measurement value M2. The memory unit 122 saves the second measurement value M2.

In step S8, the control unit 124 moves the cells in the sensor unit 34 to the bioreactor 30. For example, the control unit 124 connects the first supply flow path 56 and the medical bag of the culture medium. The control unit 124 operates the first supply pump 102 and the first circulation pump 104. According to the operations of the first supply pump 102 and the first circulation pump 104, the supply unit 22 supplies the culture medium containing no cells to the first circulation flow path 58. As a result, the cells in the sensor unit 34 flow to the bioreactor 30.

In step S9, the control unit 124 blocks the dilution flow path 132 and the first circulation flow path 58 from each other by controlling the valve 134 (Fig. 4B).

In step S10, the calibration unit 126 creates the calibration curve 140 (Fig. 6). The calibration unit 126 computes the cell concentration of the culture medium in the first circuit 136 at the time of step S5. The calibration unit 126 computes the cell concentration based on the information (volume and number of cells of the first circuit 136) saved in the memory unit 122. This cell concentration is defined as a first concentration C1. The calibration unit 126 associates the first concentration C1 with the first measurement value M1. Further, the calibration unit 126 computes the cell concentrations of the culture media of the first circuit 136 and the second circuit 138 at the time of step S7. The calibration unit 126 computes the cell concentration based on the information (volume of the first circuit 136, the volume of the second circuit 138, and number of cells of circuits) saved in the memory unit 122. This cell concentration is defined as a second concentration C2. The calibration unit 126 associates the second concentration C2 with the second measurement value M2. The cell concentration and the turbidity have a linear relationship. Therefore, the calibration unit 126 creates the calibration curve 140 of the cell concentration and the turbidity (measurement value) from the first concentration C1, the first measurement value M1, the second concentration C2, and the second measurement value M2 as illustrated in Fig. 6. The calibration unit 126 saves the calibration curve 140 in the memory unit 122. The first concentration C1 may be computed at the time of step S5, that is, when the memory unit 122 saves the first measurement value M1. In addition, the second concentration C2 may be calculated at the time of step S7, that is, at the time when the memory unit 122 saves the second measurement value M2.

According to the first embodiment, one type of cell fluid may be supplied to the cell culture circuit 16, and the cell fluid may be diluted with a dilution culture medium provided in advance in the cell culture circuit 16. According to the first embodiment, it is not necessary to prepare a plurality of concentrations of cell fluid, and it is not necessary to discard the cell fluid and the culture medium. Therefore, waste of the cell fluid and the culture medium is eliminated. Further, according to the first embodiment, calibration work is performed using the cell culture device 10. Therefore, the calibration work can be efficiently and automatically performed.

### [4 Second Embodiment]

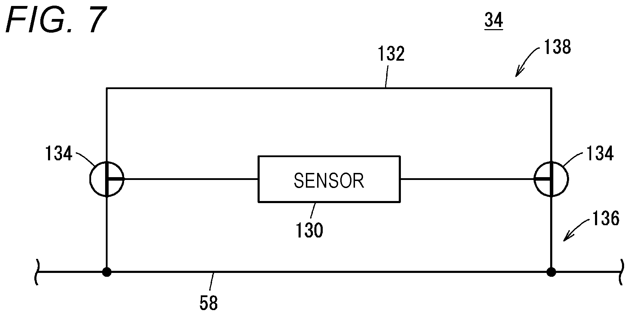

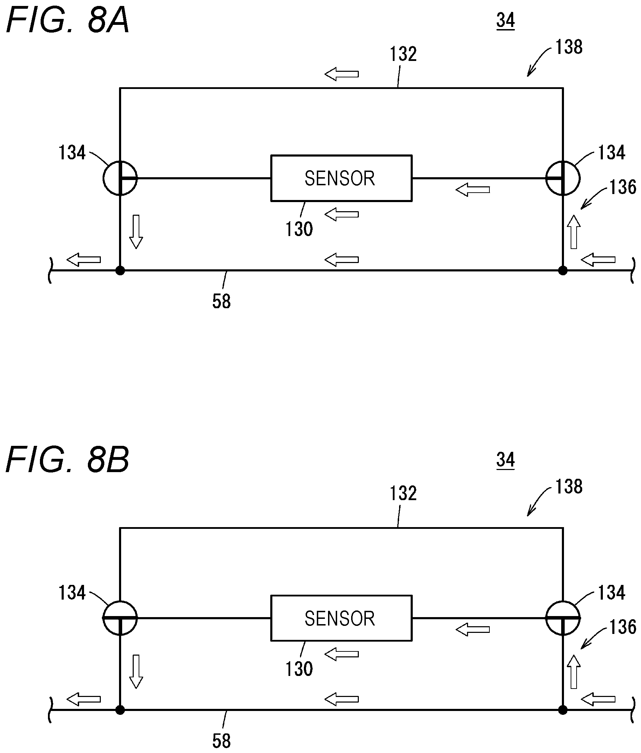

Fig. 7 is a diagram illustrating a configuration of the sensor unit 34 of a second embodiment. Figs. 8A and 8B are diagrams illustrating a connection state between the first circulation flow path 58 and the dilution flow path 132 in the sensor unit 34 of the second embodiment. In the calibration device 12 of the second embodiment, the same components as those of the calibration device 12 of the first embodiment are denoted by the same reference numerals, and the detailed description thereof will be omitted.

In the sensor unit 34 of the first embodiment, the sensor 130 is connected to the first circulation flow path 58 in series. On the other hand, in the sensor unit 34 of the second embodiment, the sensor 130 is connected to the first circulation flow path 58 in parallel. The sensor unit 34 of the second embodiment is substantially the same as the sensor unit 34 of the first embodiment except that the sensors 130 are connected in parallel.

In the second embodiment, the first circuit 136 is formed by a closed circuit including the first circulation flow path 58, the sensor 130, the bioreactor 30 (Fig. 1), and the first circulation pump 104 (Fig. 1). Furthermore, in the second embodiment, the second circuit 138 is formed by the dilution flow path 132 connected to the first circuit 136 in parallel.

The calibration processing performed by the arithmetic unit 120 in the second embodiment is the same as the calibration processing (Fig. 5) performed by the arithmetic unit 120 in the first embodiment.

The internal flow path of the sensor 130 is thin. Therefore, when the sensor 130 is connected to the first circulation flow path 58 in series as in the first embodiment, the flow rate of the culture medium flowing through the first circulation flow path 58 is affected by an increase in internal pressure in the internal flow path and the circuit of the sensor 130. On the other hand, when the sensor 130 is connected to the first circulation flow path 58 in parallel as in the second embodiment, the flow rate of the culture medium flowing through the first circulation flow path 58 is not affected by the increase in the internal pressure in the internal flow path of the sensor 130 and the circuit. Therefore, according to the second embodiment, a large volume of the cell fluid and the culture medium can flow into the first circulation flow path 58. Further, according to the second embodiment, the same effects as those of the first embodiment can be obtained.

### [5 Third Embodiment]

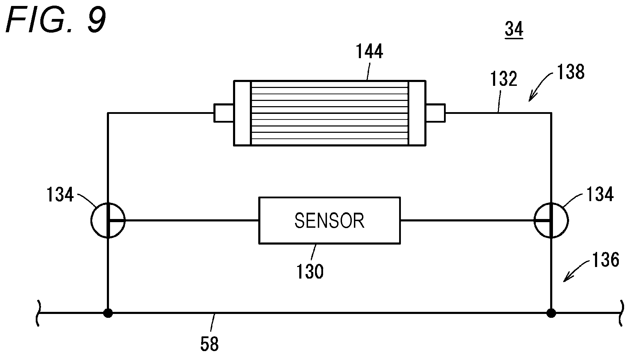

Fig. 9 is a diagram illustrating a configuration of the sensor unit 34 of a third embodiment. In the calibration device 12 of the third embodiment, the same components as those of the calibration devices 12 of the first embodiment and the second embodiment are denoted by the same reference numerals, and the detailed description thereof will be omitted.

In the third embodiment, a sub bioreactor 144 different from the bioreactor 30 is provided in the dilution flow path 132 of the second embodiment. The sub bioreactor 144 is connected to the dilution flow path 132 in series. The sub bioreactor 144 may be of the same construction as the bioreactor 30. In Fig. 9, another circulation flow path (corresponding to the second circulation flow path 62 of the bioreactor 30) connected to the sub bioreactor 144 is omitted.

In the third embodiment, the first circuit 136 is formed by a closed circuit including the first circulation flow path 58, the sensor 130, the bioreactor 30 (Fig. 1), and the first circulation pump 104 (Fig. 1). Furthermore, in the third embodiment, the second circuit 138 is formed by the dilution flow path 132 connected to the first circuit 136 in parallel and the sub bioreactor 144.

The calibration processing performed by the arithmetic unit 120 in the third embodiment is the same as the calibration processing (Fig. 5) performed by the arithmetic unit 120 in the first embodiment.

According to the third embodiment, the cells can be cultured by the bioreactor 30 and the sub bioreactor 144. Therefore, according to the third embodiment, the number of cells that can be cultured can be increased. Further, according to the third embodiment, the same effects as those of the first embodiment can be obtained.

### [6 Fourth Embodiment]

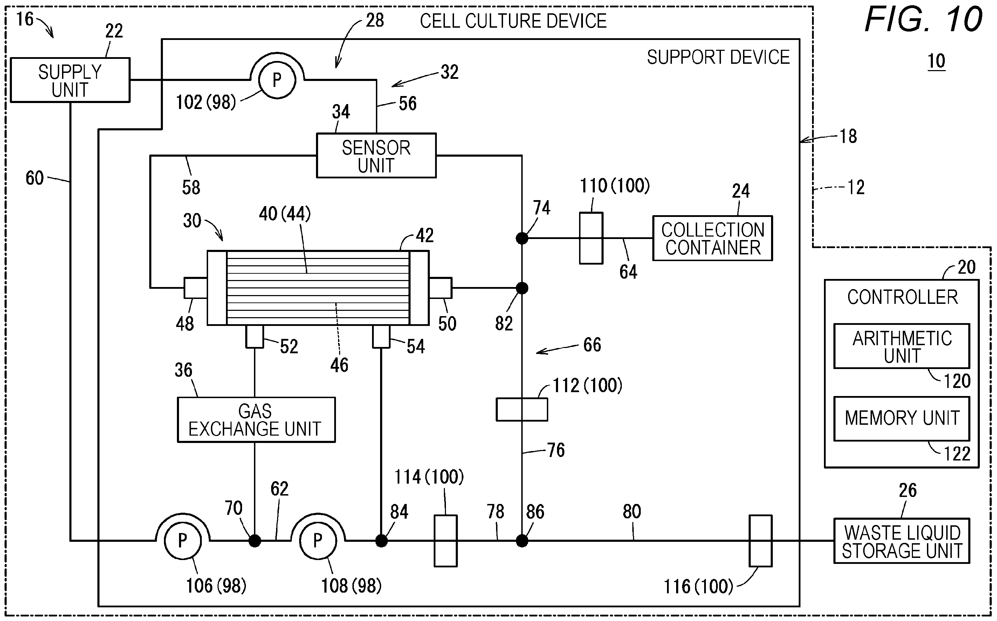

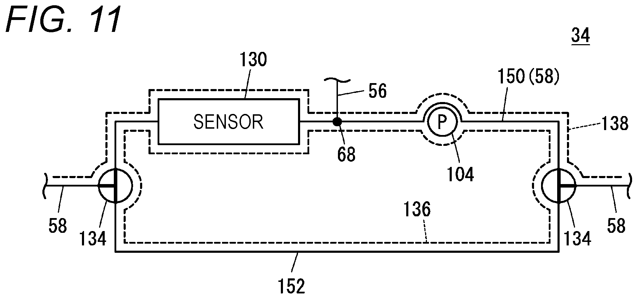

Fig. 10 is a diagram illustrating a configuration of the cell culture device 10 (calibration device 12) according to another embodiment from Fig. 1. In the cell culture device 10 of Fig. 10, the same components as those of the cell culture device 10 of Fig. 1 are denoted by the same reference numerals, and a detailed description thereof will be omitted. The cell culture device 10 in Fig. 10 is different from the cell culture device 10 in Fig. 1 in the position of the sensor unit 34. The sensor unit 34 is provided between the collection branch portion 74 of the first circulation flow path 58 and the first inlet port 48. Further, the sensor unit 34 includes the first junction portion 68 (Fig. 11).

### [6-1 Configuration of Sensor Unit 34]

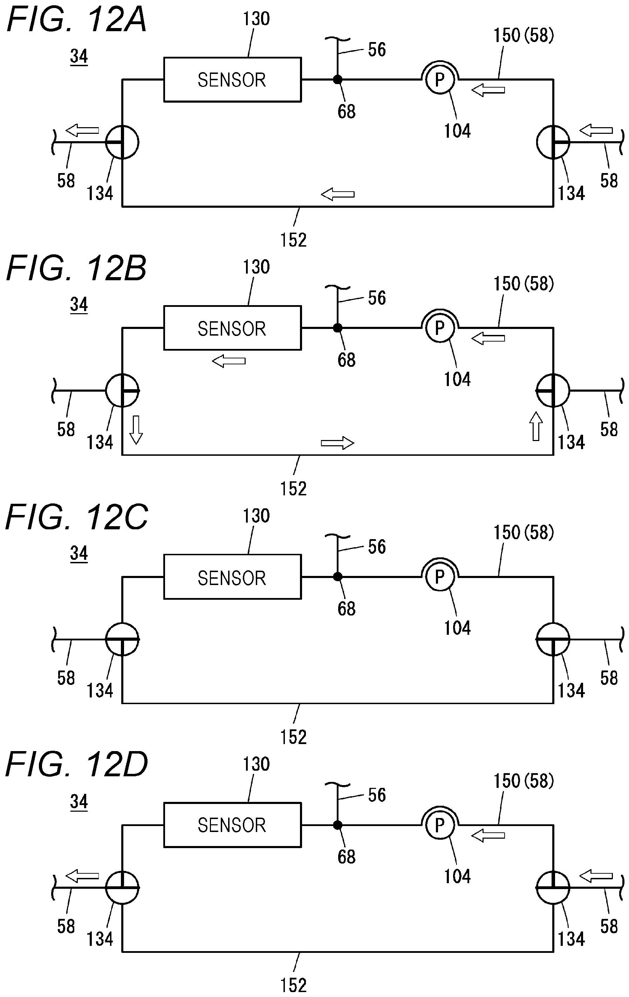

Fig. 11 is a diagram illustrating a configuration of the sensor unit 34 of a fourth embodiment. Fig. 12A to Fig. 12D are diagrams illustrating a connection state of the first circulation flow path 58, the sensor flow path 150, and the concentration flow path 152 in the sensor unit 34 of the fourth embodiment. In the fourth embodiment, the sensor unit 34 includes the sensor flow path 150, the sensor 130, the concentration flow path 152, and the two valves 134.

The sensor flow path 150 is a part of the first circulation flow path 58. The sensor flow path 150 is a portion of the first circulation flow path 58 sandwiched between the two valves 134. The sensor flow path 150 includes the first junction portion 68. Furthermore, the sensor flow path 150 is attached to the first circulation pump 104. The sensor 130 is connected to the sensor flow path 150 in series. The concentration flow path 152 is a tube member. The concentration flow path 152 is connected to the sensor flow path 150 of the first circulation flow path 58 in parallel. The volume of the first circulation flow path 58, the volume of the sensor flow path 150, the volume of the concentration flow path 152, and the volume of the bioreactor 30 are saved in the memory unit 122 in advance. The valve 134 is disposed at each connection point between the sensor flow path 150 and the concentration flow path 152.

The two valves 134 are, for example, three-way valves. As illustrated in Figs. 12A to 12D, each of the two valves 134 can switch between the communicating state and the block state between the sensor flow path 150 and the concentration flow path 152. Each of the two valves 134 can switch between a communicating state and a block state between a portion of the first circulation flow path 58 other than the sensor flow path 150 and the sensor flow path 150 (and the concentration flow path 152).

In the fourth embodiment, the first circuit 136 is formed by a closed circuit including the sensor flow path 150, the concentration flow path 152, the sensor 130, and the first circulation pump 104. In the fourth embodiment, the second circuit 138 is formed by a closed circuit including the first circulation flow path 58 (including the sensor flow path 150), the sensor 130, and the bioreactor 30 (Fig. 1).

### [6-2 Calibration Processing]

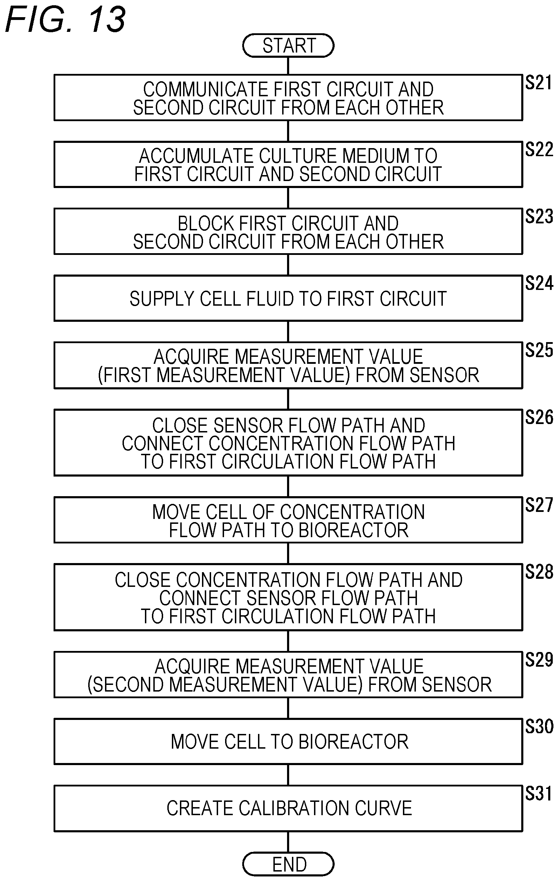

Fig. 13 is a flowchart of calibration processing according to the fourth embodiment. Before the calibration processing illustrated in Fig. 13, the user counts the number of cells in the cell fluid by a cell counter or the like. The user fills a medical bag or the like with the cell fluid of which the number of cells has been counted, and sets the medical bag or the like in the supply unit 22. In addition, the user fills the medical bag with a culture medium not containing cells, and sets the medical bag in the supply unit 22. The user saves the counted number of cells in the memory unit 122. In addition, the user fills the culture medium in the medical bag or the like, and sets the medical bag or the like in the supply unit 22.

In step S21, the control unit 124 controls the valve 134 to cause a portion of the first circulation flow path 58 other than the sensor flow path 150 and the sensor flow path 150 to communicate with each other. In addition, the control unit 124 causes the first circulation flow path 58 and the concentration flow path 152 to communicate with each other (Fig. 12A). As a result, the first circuit 136 including the sensor flow path 150 and the second circuit 138 including the sensor flow path 150 communicate with each other.

In step S22, the control unit 124 connects the first supply flow path 56 to the medical bag of the culture medium. The control unit 124 then operates the first supply pump 102 and the first circulation pump 104. According to the operations of the first supply pump 102 and the first circulation pump 104, the supply unit 22 supplies the culture medium containing no cells to the first circulation flow path 58. As a result, the culture medium not containing cells accumulates in both the first circuit 136 and the second circuit 138. After supplying the culture medium, the control unit 124 stops the first supply pump 102.

In step S23, the control unit 124 controls the valve 134 to block a portion of the first circulation flow path 58 other than the sensor flow path 150 from the sensor flow path 150. In addition, the control unit 124 blocks the first circulation flow path 58 and the concentration flow path 152 from each other. Furthermore, the control unit 124 causes the sensor flow path 150 and the concentration flow path 152 to communicate with each other (Fig. 12B). Thus, the first circuit 136 is formed. Furthermore, the first circuit 136 and the second circuit 138 are cut off from each other.

In step S24, the control unit 124 connects the first supply flow path 56 to the medical bag of the cell fluid. The control unit 124 then operates the first supply pump 102 and the first circulation pump 104. The supply unit 22 supplies the cell fluid to the sensor flow path 150 in accordance with the operations of the first supply pump 102 and the first circulation pump 104. At this point, the first circulation flow path 58 other than the sensor flow path 150 is blocked from the sensor flow path 150. Therefore, in the sensor unit 34, the cell fluid is supplied only to the first circuit 136. The control unit 124 stops the first supply pump 102 after the supply of the cell fluid is completed.

In step S25, the calibration unit 126 acquires the measurement value from the sensor 130. This measurement value is referred to as a first measurement value M1. The memory unit 122 saves the first measurement value M1.

In step S26, the control unit 124 causes the concentration flow path 152 and the first circulation flow path 58 to communicate with each other by controlling the valve 134. On the other hand, the control unit 124 blocks a portion of the first circulation flow path 58 other than the sensor flow path 150 from the sensor flow path 150. In addition, the control unit 124 blocks the sensor flow path 150 and the concentration flow path 152 from each other (Fig. 12C).

In step S27, the control unit 124 moves the cells in the sensor unit 34 to the bioreactor 30. As in step S8 in Fig. 5, the control unit 124 operates the first supply pump 102 and the first circulation pump 104 to supply the culture medium not containing cells from the supply unit 22 to the first circulation flow path 58.

In step S28, the control unit 124 controls the valve 134 to cause a portion of the first circulation flow path 58 other than the sensor flow path 150 and the sensor flow path 150 to communicate with each other. In addition, the control unit 124 blocks the first circulation flow path 58 and the concentration flow path 152 from each other. Furthermore, the control unit 124 blocks the sensor flow path 150 and the concentration flow path 152 from each other (Fig. 12D). Thus, the second circuit 138 is formed. Then, the culture medium other than the sensor flow path 150 in the first circulation flow path 58 and the culture medium in the sensor flow path 150 are mixed. As a result, the culture medium containing the cells is diluted.

In step S29, the calibration unit 126 acquires the measurement value from the sensor 130. This measurement value is referred to as a second measurement value M2. The memory unit 122 saves the second measurement value M2.

In step S30, the control unit 124 moves the cells contained in the culture medium to the bioreactor 30.

In step S31, the calibration unit 126 creates the calibration curve 140 (Fig. 6). The calibration unit 126 computes the cell concentration of the culture medium in the first circuit 136 at the time of step S25. The calibration unit 126 computes the cell concentration based on the information (volume and number of cells of the first circuit 136) saved in the memory unit 122. This cell concentration is defined as a first concentration C1. The calibration unit 126 associates the first concentration C1 with the first measurement value M1. Further, the calibration unit 126 calculates the cell concentration of the culture medium in the second circuit 138 at the time of step S29. The calibration unit 126 computes the cell concentration based on the information (volume and number of cells of the second circuit 138) saved in the memory unit 122. This cell concentration is defined as a second concentration C2. The calibration unit 126 associates the second concentration C2 with the second measurement value M2. The cell concentration and the turbidity have a linear relationship. Therefore, the calibration unit 126 creates the calibration curve 140 of the cell concentration and the turbidity (measurement value) from the first concentration C1, the first measurement value M1, the second concentration C2, and the second measurement value M2 as illustrated in Fig. 6. The calibration unit 126 saves the calibration curve 140 in the memory unit 122. The first concentration C1 may be computed at the time of step S25, that is, when the memory unit 122 saves the first measurement value M1. In addition, the second concentration C2 may be calculated at the time of step S29, that is, at the time when the memory unit 122 saves the second measurement value M2.

According to the fourth embodiment, the same effects as those of the first embodiment can be obtained. Furthermore, according to the fourth embodiment, more accurate calibration can be performed by measuring the minimum concentration at the time of cell input and the maximum concentration in the cell culture device 10.

### [7 Fifth Embodiment]

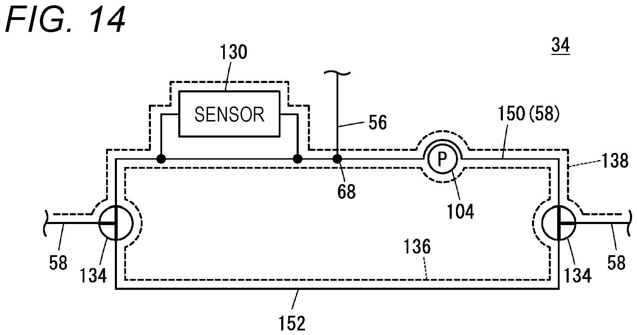

Fig. 14 is a diagram illustrating a configuration of the sensor unit 34 of a fifth embodiment. In the calibration device 12 of the fifth embodiment, the same components as those of the calibration device 12 of the fourth embodiment are denoted by the same reference numerals, and the detailed description thereof will be omitted.

In the sensor unit 34 of the fourth embodiment, the sensor 130 is connected to the sensor flow path 150 in series. On the other hand, in the sensor unit 34 of the fifth embodiment, the sensor 130 is connected to the sensor flow path 150 in parallel. The sensor unit 34 of the fifth embodiment is substantially the same as the sensor unit 34 of the fourth embodiment except that the sensors 130 are connected in parallel.

In the fifth embodiment, the first circuit 136 is formed by a closed circuit including the sensor flow path 150, the concentration flow path 152, the sensor 130, and the first circulation pump 104. In the fifth embodiment, the second circuit 138 is formed by a closed circuit including the first circulation flow path 58 (including the sensor flow path 150), the sensor 130, and the bioreactor 30.

The calibration processing performed by the arithmetic unit 120 in the fifth embodiment is the same as the calibration processing (Fig. 13) performed by the arithmetic unit 120 in the fourth embodiment.

When the sensor 130 is connected to the sensor flow path 150 in parallel as in the fifth embodiment, the flow rate of the culture medium flowing through the sensor flow path 150 is not affected by an increase in internal pressure in the internal flow path and the circuit of the sensor 130. Therefore, according to the fifth embodiment, a large volume of the cell fluid and the culture medium can flow through the sensor flow path 150 (first circulation flow path 58). Further, according to the fifth embodiment, the same effects as those of the fourth embodiment can be obtained.

### [8 Sixth Embodiment]

### [8-1 Configuration of Sensor Unit 34]

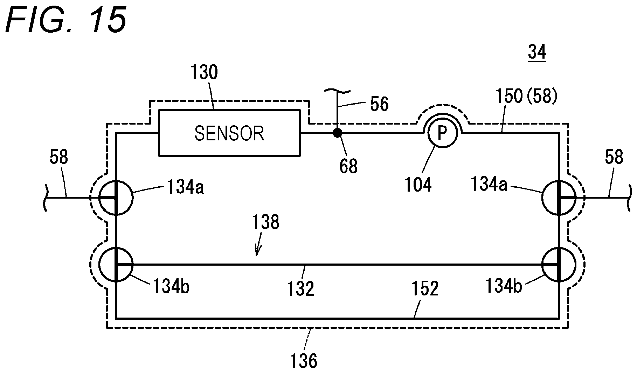

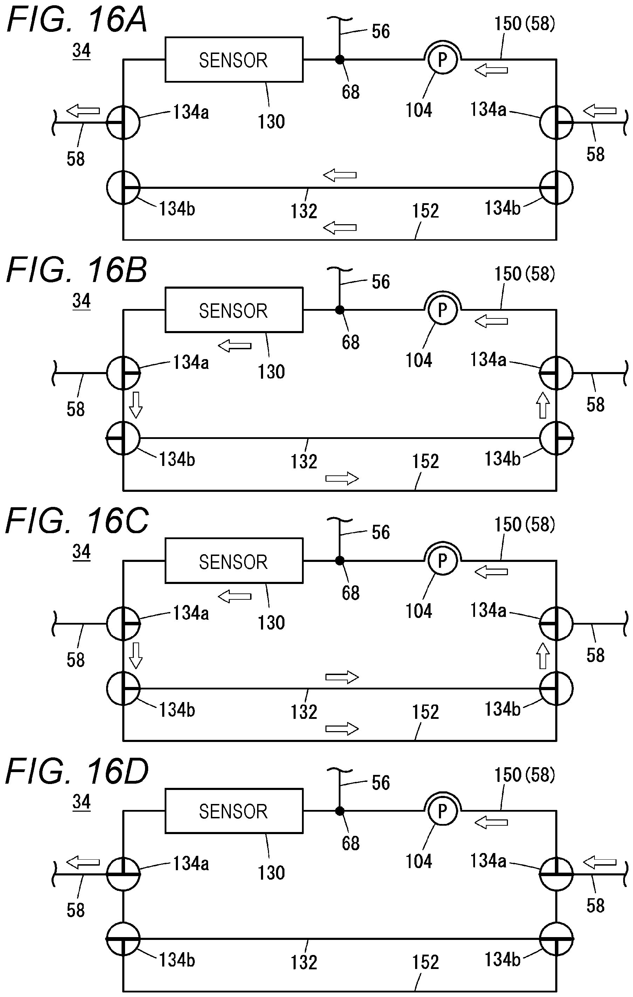

Fig. 15 is a diagram illustrating a configuration of the sensor unit 34 of a sixth embodiment. Fig. 16A to Fig. 16D are diagrams illustrating a connection state between the first circulation flow path 58, the sensor flow path 150, the concentration flow path 152, and the dilution flow path 132 in the sensor unit 34 of the sixth embodiment. In the calibration device 12 of the sixth embodiment, the same components as those of the calibration devices 12 of the fourth embodiment and the fifth embodiment are denoted by the same reference numerals, and the detailed description thereof will be omitted. In the sixth embodiment, the sensor unit 34 includes the sensor flow path 150, the sensor 130, the concentration flow path 152, the dilution flow path 132, two first valves 134a, and two second valves 134b.

The sixth embodiment is an embodiment in which the first embodiment and the fourth embodiment are combined. In the sixth embodiment, the calibration processing is performed using a closed circuit not including the bioreactor 30.

The dilution flow path 132 is a tube member. The dilution flow path 132 is connected to the concentration flow path 152 in parallel. The volume of the sensor flow path 150, the volume of the concentration flow path 152, and the volume of the dilution flow path 132 are saved in the memory unit 122 in advance. The first valve 134a is disposed at each connection point between the sensor flow path 150 and the concentration flow path 152. The second valve 134b is disposed at each connection point between the concentration flow path 152 and the dilution flow path 132.

The two first valves 134a and the two second valves 134b are, for example, three-way valves. As illustrated in Figs. 16A to 16D, each of the two first valves 134a and the two second valves 134b can switch between a communication state and a cutoff state of the sensor flow path 150, the concentration flow path 152, and the dilution flow path 132.

In the sixth embodiment, the first circuit 136 is formed by a closed circuit including the sensor flow path 150, the concentration flow path 152, the sensor 130, and the first circulation pump 104. Furthermore, in the sixth embodiment, the second circuit 138 is formed by the dilution flow path 132 connected to the first circuit 136 in parallel.

### [8-2 Calibration Processing]

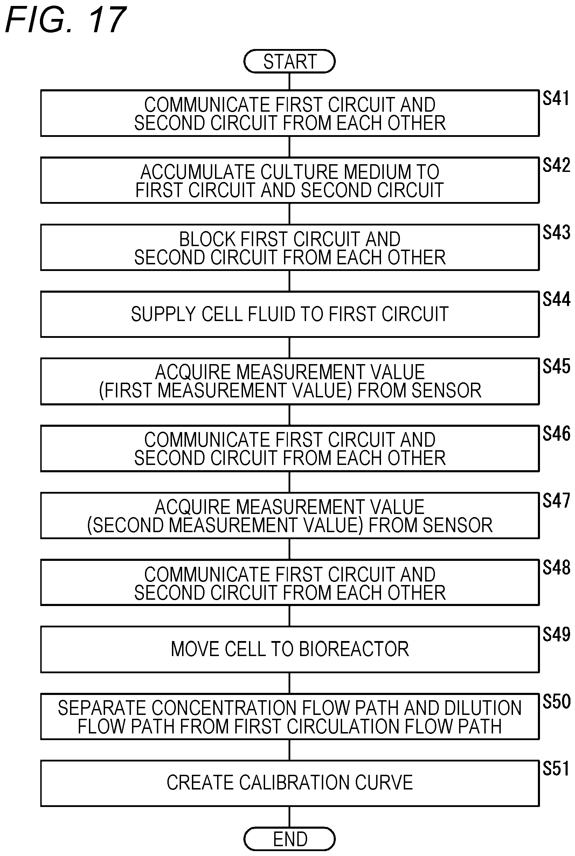

Fig. 17 is a flowchart of calibration processing according to the sixth embodiment. Before the calibration processing illustrated in Fig. 17, the user counts the number of cells in the cell fluid by a cell counter or the like. The user fills a medical bag or the like with the cell fluid of which the number of cells has been counted, and sets the medical bag or the like in the supply unit 22. In addition, the user fills the medical bag with a culture medium not containing cells, and sets the medical bag in the supply unit 22. The user saves the counted number of cells in the memory unit 122. In addition, the user fills the culture medium in the medical bag or the like, and sets the medical bag or the like in the supply unit 22.

In step S41, the control unit 124 controls the first valve 134a and the second valve 134b. The control unit 124 causes a portion of the first circulation flow path 58 other than the sensor flow path 150 and the sensor flow path 150 to communicate with each other. In addition, the control unit 124 causes the first circulation flow path 58 and the concentration flow path 152 to communicate with each other. Further, the control unit 124 causes the first circulation flow path 58 and the dilution flow path 132 to communicate with each other. Furthermore, the control unit 124 causes the sensor flow path 150 and the concentration flow path 152 to communicate with each other. In addition, the control unit 124 causes the sensor flow path 150 (and the concentration flow path 152) and the dilution flow path 132 to communicate with each other (Fig. 16A). As a result, the first circuit 136 and the second circuit 138 communicate with each other.

In step S42, the control unit 124 connects the first supply flow path 56 to the medical bag of the culture medium. The control unit 124 then operates the first supply pump 102 and the first circulation pump 104. According to the operations of the first supply pump 102 and the first circulation pump 104, the supply unit 22 supplies the culture medium containing no cells to the first circulation flow path 58. As a result, the culture medium not containing cells accumulates in both the first circuit 136 and the second circuit 138. After supplying the culture medium, the control unit 124 stops the first supply pump 102.

In step S43, the control unit 124 controls the first valve 134a and the second valve 134b. The control unit 124 blocks a portion of the first circulation flow path 58 other than the sensor flow path 150 from the sensor flow path 150. In addition, the control unit 124 blocks a portion of the first circulation flow path 58 other than the sensor flow path 150 from the concentration flow path 152. In addition, the control unit 124 blocks a portion of the first circulation flow path 58 other than the sensor flow path 150 and the dilution flow path 132 from each other. Furthermore, the control unit 124 causes the sensor flow path 150 and the concentration flow path 152 to communicate with each other. In addition, the control unit 124 blocks the sensor flow path 150 (and the concentration flow path 152) and the dilution flow path 132 from each other (Fig. 16B). Thus, the first circuit 136 is formed. Furthermore, the first circuit 136 and the second circuit 138 are cut off from each other.

In step S44, the control unit 124 connects the first supply flow path 56 to the medical bag of the cell fluid. The control unit 124 then operates the first supply pump 102 and the first circulation pump 104. The supply unit 22 supplies the cell fluid to the sensor flow path 150 in accordance with the operations of the first supply pump 102 and the first circulation pump 104. At this point, the first circulation flow path 58 other than the sensor flow path 150 is blocked from the sensor flow path 150. Therefore, in the sensor unit 34, the cell fluid is supplied only to the first circuit 136. The control unit 124 stops the first supply pump 102 after the supply of the cell fluid is completed.

In step S45, the calibration unit 126 acquires the measurement value from the sensor 130. This measurement value is referred to as a first measurement value M1. The memory unit 122 saves the first measurement value M1.

In step S46, the control unit 124 causes the sensor flow path 150 (and the concentration flow path 152) and the dilution flow path 132 to communicate with each other by controlling the second valve 134b (Fig. 16C). As a result, the first circuit 136 and the second circuit 138 communicate with each other. Then, the culture medium of the second circuit 138 and the culture medium of the first circuit 136 are mixed. As a result, the culture medium containing the cells is diluted.

In step S47, the calibration unit 126 acquires the measurement value from the sensor 130. This measurement value is referred to as a second measurement value M2. The memory unit 122 saves the second measurement value M2.

In step S48, the control unit 124 controls the first valve 134a and the second valve 134b. The control unit 124 brings the respective flow paths into the same communication state as in step S1 (Fig. 16A). As a result, the first circuit 136 and the second circuit 138 communicate with each other.

In step S49, the control unit 124 moves the cells in the sensor unit 34 to the bioreactor 30. As in step S8 in Fig. 5, the control unit 124 operates the first supply pump 102 and the first circulation pump 104 to supply the culture medium not containing cells from the supply unit 22 to the first circulation flow path 58.

In step S50, the control unit 124 controls the first valve 134a and the second valve 134b. The control unit 124 causes a portion of the first circulation flow path 58 other than the sensor flow path 150 and the sensor flow path 150 to communicate with each other. In addition, the control unit 124 blocks the first circulation flow path 58 and the concentration flow path 152 from each other. In addition, the control unit 124 blocks the first circulation flow path 58 and the dilution flow path 132 from each other (Fig. 16D). As a result, the concentration flow path 152 and the dilution flow path 132 are separated from the first circulation flow path 58.

In step S51, the calibration unit 126 creates the calibration curve 140 (Fig. 6). The calibration unit 126 computes the cell concentration of the culture medium in the first circuit 136 at the time of step S45. The calibration unit 126 computes the cell concentration based on the information (volume and number of cells of the first circuit 136) saved in the memory unit 122. This cell concentration is defined as a first concentration C1. The calibration unit 126 associates the first concentration C1 with the first measurement value M1. Further, the calibration unit 126 calculates the cell concentration of the culture medium in the second circuit 138 at the time of step S47. The calibration unit 126 computes the cell concentration based on the information (volume of the first circuit 136, the volume of the second circuit 138, and number of cells of circuits) saved in the memory unit 122. This cell concentration is defined as a second concentration C2. The calibration unit 126 associates the second concentration C2 with the second measurement value M2. The cell concentration and the turbidity have a linear relationship. Therefore, the calibration unit 126 creates the calibration curve 140 of the cell concentration and the turbidity (measurement value) from the first concentration C1, the first measurement value M1, the second concentration C2, and the second measurement value M2 as illustrated in Fig. 6. The calibration unit 126 saves the calibration curve 140 in the memory unit 122. The first concentration C1 may be computed at the time of step S45, that is, when the memory unit 122 saves the first measurement value M1. In addition, the second concentration C2 may be calculated at the time of step S47, that is, at the time when the memory unit 122 saves the second measurement value M2.

According to the sixth embodiment, both the first circuit 136 and the second circuit 138 do not include the bioreactor 30. Therefore, according to the sixth embodiment, the calibration processing can be performed in a short time. Further, according to the sixth embodiment, the same effects as those of the fourth embodiment can be obtained.

### [9 Invention obtained from Embodiments]

The invention that can be grasped from the above embodiments will be described below.

A first aspect of the present invention is a cell culture device (10) including a cell culture circuit (16) capable of circulating a culture medium to which a cell fluid is supplied, a sensor (130) that is disposed in the cell culture circuit and measures turbidity of the cell fluid, and a calibration unit (126) that calibrates a measurement value of the sensor to a cell concentration of the culture medium. The sensor measures, as a first measurement value (M1), the turbidity of a first volume of the culture medium in which the cell fluid containing a predetermined number of cells is supplied, and measures, as a second measurement value (M2), the turbidity of the culture medium generated by diluting the first volume of the culture medium with a second volume of the culture medium not containing cells. The calibration unit calculates the cell concentration of the culture medium in which the cell fluid is supplied as a first concentration (C1), calculates the cell concentration of the culture medium after dilution as a second concentration (C2), associates the first concentration with the first measurement value, and associates the second concentration with the second measurement value, thereby creating a calibration curve (140) between the measurement value of the sensor and the cell concentration.

In the first aspect, the cell culture device may include a supply unit (22) that supplies the cell fluid to the cell culture circuit, and a control unit (124) that controls the cell culture circuit. The cell culture circuit may include a first circuit (136) forming a closed circuit including the sensor, a second circuit (138) connected in parallel to the first circuit, and a plurality of valves (134, 134a, and 134b) disposed at respective connection points between the first circuit and the second circuit. The control unit may control each of the valves to block the first circuit and the second circuit from each other in a state where the first circuit and the second circuit are filled with the culture medium not containing cells, and after the cell fluid containing a predetermined number of cells is supplied to the first circuit by the supply unit, the control unit may control the valve to cause the first circuit and the second circuit to communicate with each other, thereby diluting the culture medium of the first circuit with the culture medium of the second circuit.

In the first aspect, the first circuit may include a bioreactor (30) and a pump (104).

In the first aspect, the first circuit may include the first bioreactor (30) and the pump, and the second circuit may include a second bioreactor (144).

In the first aspect, the sensor may be connected to the closed circuit in parallel.

In the first aspect, the cell culture device may include a sensor flow path (150) in which the sensor is disposed, a supply unit that supplies the cell fluid to the cell culture circuit, and a control unit that controls the cell culture circuit. The cell culture circuit may include a first circuit capable of forming a closed circuit including the sensor flow path, a second circuit connected in parallel to the first circuit and capable of forming a closed circuit including the sensor flow path, and a plurality of valves disposed at respective connection points between the first circuit and the second circuit. In a state where the first circuit and the second circuit are filled with the culture medium not containing cells, the control unit may control each of the valves to form the first circuit including the sensor flow path and block the first circuit and the second circuit from each other, and after the cell fluid containing a predetermined number of cells is supplied to the first circuit by the supply unit, the control unit may control the valve to form the second circuit including the sensor flow path and block the first circuit and the second circuit from each other to dilute the culture medium of the sensor flow path with the culture medium of the second circuit.

In the first aspect, the sensor flow path may include a pump, and the second circuit may include a bioreactor.

A second aspect of the present invention is a calibration method using a cell culture circuit capable of circulating a culture medium to which a cell fluid is supplied, a sensor that is arranged in the cell culture circuit and measures turbidity of the cell fluid, and a calibration unit that calibrates a measurement value of the sensor to a cell concentration of the culture medium. The sensor measures, as a first measurement value, the turbidity of a first volume of the culture medium in which the cell fluid containing a predetermined number of cells is supplied, and measures, as a second measurement value, the turbidity of the culture medium generated by diluting the first volume of the culture medium with a second volume of the culture medium not containing cells. The calibration unit calculates the cell concentration of the culture medium in which the cell fluid is supplied as a first concentration, calculates the cell concentration of the culture medium after dilution as a second concentration, associates the first concentration with the first measurement value, and associates the second concentration with the second measurement value, thereby creating a calibration curve between the measurement value of the sensor and the cell concentration.

## Claims

1. A cell culture device comprising:
a cell culture circuit capable of circulating a culture medium in which a cell fluid is supplied;
a sensor that is disposed in the cell culture circuit and measures turbidity of the cell fluid; and
a calibration unit that calibrates the measurement value of the sensor to a cell concentration of the culture medium, wherein
the sensor measures, as a first measurement value, the turbidity of a first volume of the culture medium in which the cell fluid containing a predetermined number of cells is supplied, and measures, as a second measurement value, the turbidity of the culture medium generated by diluting the first volume of the culture medium with a second volume of the culture medium not containing cells, and
the calibration unit calculates, as a first concentration, the cell concentration of the culture medium in which the cell fluid is supplied, calculates, as a second concentration, the cell concentration of the culture medium after dilution, and creates a calibration curve between the measurement value of the sensor and the cell concentration by associating the first concentration with the first measurement value and associating the second concentration with the second measurement value.

2. The cell culture device according to claim 1, further comprising:
a supply unit that supplies the cell fluid to the cell culture circuit; and
a control unit that controls the cell culture circuit, wherein
the cell culture circuit includes
a first circuit that forms a closed circuit including the sensor,
a second circuit connected to the first circuit in parallel, and
a plurality of valves disposed at respective connection points between the first circuit and the second circuit, and
the control unit controls,
in a state where the first circuit and the second circuit are filled with the culture medium containing no cells, each of the valves to block the first circuit and the second circuit from each other, and
controls, after the cell fluid containing a predetermined number of cells is supplied to the first circuit by the supply unit, the valve to communicate the first circuit and the second circuit with each other, thereby diluting the culture medium of the first circuit with the culture medium of the second circuit.

3. The cell culture device according to claim 2, wherein the first circuit includes a bioreactor and a pump.

4. The cell culture device according to claim 2, wherein
the first circuit includes a first bioreactor and a pump, and
the second circuit includes a second bioreactor.

5. The cell culture device according to any one of claims 2 to 4, wherein
the sensor is connected to the closed circuit in parallel.

6. The cell culture device according to claim 1, further comprising:
a sensor flow path in which the sensor is disposed;
a supply unit that supplies the cell fluid to the cell culture circuit; and
a control unit that controls the cell culture circuit, wherein
the cell culture circuit includes
a first circuit capable of forming a closed circuit including the sensor flow path,
a second circuit connected to the first circuit in parallel and capable of forming a closed circuit including the sensor flow path, and
a plurality of valves disposed at respective connection points between the first circuit and the second circuit, and
the control unit controls,
in a state where the first circuit and the second circuit are filled with the culture medium containing no cells, each of the valves to form the first circuit including the sensor flow path, and block the first circuit and the second circuit from each other, and
controls, after the supply unit supplies the cell fluid containing a predetermined number of cells to the first circuit, the valve to form the second circuit including the sensor flow path, and block the first circuit and the second circuit from each other, thereby diluting the culture medium of the sensor flow path with the culture medium of the second circuit.

7. The cell culture device according to claim 6, wherein
the sensor flow path includes a pump, and
the second circuit includes a bioreactor.

8. A calibration method using
a cell culture circuit capable of circulating a culture medium in which a cell fluid is supplied,
a sensor that is disposed in the cell culture circuit and measures turbidity of the cell fluid, and
a calibration unit that calibrates the measurement value of the sensor to a cell concentration of the culture medium, the method comprising:
causing the sensor to measure, as a first measurement value, the turbidity of a first volume of the culture medium in which the cell fluid containing a predetermined number of cells is supplied, and measure, as a second measurement value, the turbidity of the culture medium generated by diluting the first volume of the culture medium with a second volume of the culture medium not containing cells; and
causing the calibration unit to calculate, as a first concentration, the cell concentration of the culture medium in which the cell fluid is supplied, calculate, as a second concentration, the cell concentration of the culture medium after dilution, and create a calibration curve between the measurement value of the sensor and the cell concentration by associating the first concentration with the first measurement value and associating the second concentration with the second measurement value.
